# EUROPEAN PATENT APPLICATION

(11) **EP 1 589 143 A1**
(43) Date of publication of application: **26.10.2005**
(21) Application number: 04101610.6
(22) Date of filing: 19.04.2004
(51) Int. Cl.: D06F 37/26

(54) **Household appliance for washing and/or drying clothes with a bellow comprising a fragrance compound**

(71) Applicant: Electrolux Home Products Corporation N.V., 1930 Zaventem (BE)
(72) Inventor: Colombera, Giovanni, 33077, Sacile (PN) (IT)
(74) Representative: Giugni, Valter

(57) **Abstract**

Household appliance for washing and/or drying clothes comprising an outer cabinet (2) provided with an opening (8) for loading and unloading clothes and a door (9) for closing the opening (8), a drum (14) rotatably supported inside said cabinet (2), an opening (19) provided in said drum (14) in correspondence to the opening (8) of the cabinet (2), and a flexible member (18) connecting the opening (8) of the cabinet (2) to the opening (19) of the drum (14) with watertightness. The flexible member (18) comprises a polymeric material and at least one fragrance compound mixed in at least a portion of said polymeric material.

## Description

The present invention relates to a clothes washing machine, or a similar appliance such as a washer and/or dryer, particularly intended for a household utilisation.

As is well known, a machine of this type includes an outer cabinet provided with an opening for a front or an upper loading door to load and unload the clothes to be washed and tumbled in and out of a shallow pool of water. To accomplish this washing and tumbling an inner tub contains the washing solution and a rotating drum. During operation, a lower portion of both the stationary tub and the rotating drum are filled with water.

To prevent water within the rotating drum from escaping from the inner tub, a sealing gasket or bellows is positioned between the opening provided in the outer cabinet for the loading door and an associated opening provided in the inner tub. It is also generally known, that the bellows has a plurality of peripheral circumvolutions along a substantial portion of the bellows length in order to direct any water that escapes to an appropriate location and let the inner tub oscillate with respect to the outer cabinet when the machine is operated. The bellows also include, integrally made with the said circumvolutions, a first and a second flanged end. The former end is secured to the opening provided in the outer cabinet for the loading door and the latter end is secured to the opening provided in the inner tub.

The known bellows need to be made with a material, which is a mixture having elastomeric properties, able to meet various or even opposing requirements. In particular, such a material has to meet the severe requirements imposed by the latter mentioned end of the bellows. The end of the bellows secured to the loading opening in the inner tub must resist to the repeated abrasions, caused by contact with clothes kept in movement within the rotary drum, and to the corrosion due to the washing solution. At the same time, the bellows must have a good dimensional stability to guarantee a seal against water leakage. As a consequence, it is a common practice for the manufacturers of washing machines to make the entire bellows as a single piece with synthetic polymeric material of proper hardness, so as to satisfy all of the above mentioned requirements.

However in these types of household appliances is generally felt the problem associated to the bad smell emitted by the bellows due to the material employed for its manufacture. Such bad smell or malodour is produced by polymeric materials that constitute the bellows and by specific synthetic components added in order to increase the mechanical resistance of the bellows during high temperature working. When using synthetic polymeric material, the smell from such material is very undesirable, especially in presence of wet and humidity condition. In contrast, people generally enjoy fragrances and pleasant smells.

It is to be noted that in the last years the dimension of the port-hole in the washing machine and the like is increased to facilitate the loading and unloading of the laundry. Accordingly the size of the bellows is enhanced together with the amount of polymeric material forming the bellows and hence the malodour emitted. The malodour is particularly unpleasant when the household appliance is opened the first time after being packed for a period of time as it occurs at sales.

The object of the present invention is therefore to solve the noted problem, eliminating the drawbacks of the cited known art and thus providing a bellows which are of a simple, reliable and low cost construction, and can also be easily installed.

According to the present invention, this aim is reached in a flexible member having the characteristics as recited and defined in the appended claims.

Anyway, features and advantages of the present invention may be more readily understood from the description that is given below by way of a non-limiting example with reference to the accompanying drawing, in which:

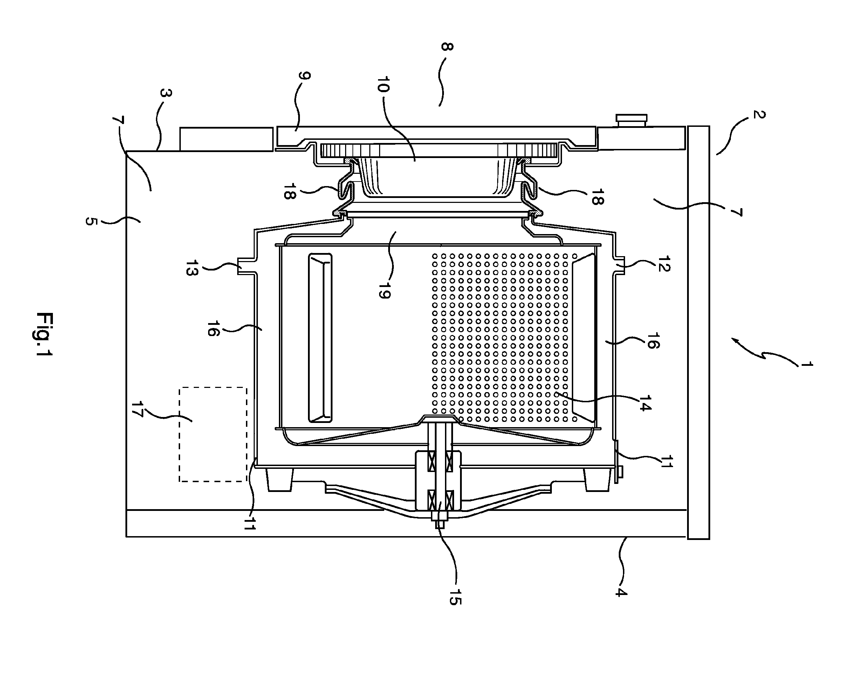

- Figure 1 is a side cross-sectional view of a household appliance according to the present invention;

As is well known in the art, a household appliance 1 for washing and/or drying clothes has an outer cabinet 2, which may be constructed of a plurality of panels attached to a frame. Referring to Fig. 1, in which is shown for illustrative purpose a front loading washing machine, the panels may include a front panel 3, a rear panel 4, side panels 5 and a worktop panel 6. These panels cooperate to define a hollow space 7. The front panel 3 is provided with an opening 8 to provide access to the hollow space 7. The opening 8 may be located at any height from the supporting surface on which, the washing machine rests. In general, the opening 8 is located near the middle portion of the front panel 3, slightly above it allowing water to be held within the machine. The opening 8 may be generally circular in shape. A door 9 may be hinged mounted on front panel 3. In case of a front loading machine, as in the presently described embodiment, the door for loading and unloading the clothes is in the form of a so-called port-hole, pivoted onto the front panel 3. The door 9 prevents clothing, water, and detergent from being thrown from the washing machine. The door 9 typically provides a broad annular front, and an inwardly or rearwardly sloping back of lesser diameter and finally, on some models, a transparent window 10 to allow the user to view the washing operation. As shown in fig. 1, a so-called washing unit is set inside the outer cabinet 2 and it comprises a stationary tub 11 for the washing liquid mounted within the hollow space 7, and provided with at least one inlet 12 for fluid supply and at least one outlet 13 along the lower portion thereof. Both inlet and outlet are provided with means to selectively control the fluid flow into and out of the stationary tub 11, respectively. A perforated drum 14is rotatably mounted within and concentrically aligned with stationary tub 11 and constitutes a washing basket within which the articles of clothing are laundered. The rotating drum 14 may be mounted on a shaft 15 located on the outer surface of rotating drum 14. This shaft 15 penetrates the stationary tub 11, and is supported by suitable bearings. A predetermined amount of clearance 16 is provided between the tub 11 and the drum 14 so as to allow the rotating drum 14 to be driven by a motor 17. Although not shown, the motor 17, preferably constituted by a variable speed, reversible electric motor, is mounted within the cabinet 2, in the present embodiment beneath the stationary tub 11, in a suitable manner. The motor 17 drives the rotating drum 14 via a suitable connection, for example a belt and pulley (not shown). More specifically, the drum 14 is rotated during both wash and rinse cycles such that articles of clothing placed therein actually tumble through either water, water/detergent or another washing fluid supplied within the drum 14. Given that the drum 14 is provided with at least a plurality of perforations that fluidly communicate with stationary tub 11, the water or water/detergent can flow between the drum 14 and the tub 11. A pumping system (not shown) is provided to control the level of washing fluid within machine, with one pump particularly controlling the timed draining of the fluid from the tub 11. When in operation, the stationary tub 11 fills with a shallow pool of water, the clothes within rotating drum 14 are tumbled through this shallow pool, and water from the pool passes into the clothes through the perforations. The stationary tub 11 is closed at the rear and is open at the front, which is formed by short, radially inwardly directed face.

To prevent water from entering the hollow space 7, a flexible annular member as a bellows, designated by the general reference 18, is positioned between the opening 8 in the outer cabinet 2 for the port-hole 10 and the stationary tub 11 in correspondence to an opening 19 of the drum 14.

Stationary tub 11 and front panel 3 are each provided with annular channels and, respectively, for attachment of bellows 18.

It can be easily appreciate that in the case of a upper loading washing machine the ends of the flexible member will be square rather than circular, as it is well known to those skilled in the art.

The flexible member 18 is made with a synthetic polymeric material either elastomeric or plastomeric having a hardness of about Shore A 25-55 and resistant to temperatures up to about 160-170 degrees Celsius. The relatively low elastic modulus allows the flexible member 18 to be particularly efficient in dampening the vibrations transmitted from the inner washing unit to the outer cabinet 2 of the washing machine. Further due to the mechanical properties of the polymer, the flexible member 18 has a high resistance to wear (rubbing action by the clothes) and to corrosion (alkaline titration of the washing solution).

According to the present invention at least one fragrance compound is mixed in at least a portion of said polymeric material to obtain a flexible member 18 emitting a stable fragrance uniformly for a long period. Either natural or synthetic fragrance compound may be used in the present invention. Examples of natural fragrance compound are animal or vegetable perfumes such as lavender oil, citronella oil, rose oil, lemon oil and jasmin oil. Examples of synthetic fragrance compound are ethyl acetoacetate, acetophenone, anisic aldehyde, benzyl benzoate, amyl cinnamic aldehyde, methyl benzoate, ethyl vanillin, ethylene brassylate, citronellyl formate, coumarin , cinnamyl alcohol, citronellyl acetate, terpinyl acetate, benzyl acetate, isoamyl salicylate, benzyl salicylate, cyclamen aldehyde, citral, itronellol, tetrahydrolinalool, ethyl phenylacetate, heliotropin, musk ambrette, p-methylacetophenone, methylionone, ethyl methyl phenylglycidate, 1-menthol, linonene & dipentene, and rosinol.

It is also possible to use a mixture of two or more kinds of fragrance compounds, if required.

The flexible member 18 is manufactured providing a polymeric material, moulding said polymeric material, adding at least one fragrance compound into said polymeric material during said moulding phase and curing said material with said at least one fragrance therein. Injection or pressure moulding could be used as well as an extrusion moulding.

The polymeric material may comprise a thermoplastic and/or thermosetting resin.

The fragrance compound is employed in any suitable percentage by weight of the total mass of the flexible member 18 according to the type of polymeric material utilised so that the flexible member emits stable aroma continuously for a certain length of time period, i.e. the bellows produces a permanent emission of aroma. The aroma produced by the flexible member 18 due to the fragrance compound is diffused into air only slowly maintaining the aroma for a long period of time. The rate at which the aroma is diffused can be controlled easily if the type of fragrance compound and its amount are appropriately varied.

According to a further embodiment of the present invention the flexible member 18, made with a synthetic polymeric material, is adapted to removably house a tablet including at least one fragrance compound either natural or synthetic. For this purpose the flexible member 18 comprises at least one pocket for removably housing the tablet but also other equivalent type of housing, known in the art, can be provided.

The pocket is provided in correspondence to the opening 8 of the outer cabinet 2. Particularly in a front loading machine the tablet is arranged at an upper portion of the flexible member 18 in the proximity of the opening 8 of the outer cabinet 2.

Advantageously the tablet comprises a polymeric material and at least one fragrance compound mixed in at least a portion of said polymeric material.

Conclusively, it can therefore be stated that the flexible member according to the present invention reaches the above-mentioned object, thereby doing away with the serious drawback shared by prior-art household appliance.

The aroma emitted by the fragrance compound cover the malodour due to the synthetic components which forms the flexible member and it can be diffused in the household appliance and in its close surrounding of the household appliance.

Further the aroma cover the bad smell coming from water stagnation occurring in the tub or in the drain circuit when the household appliance stayed idle for a long period.

## Claims

1. Household appliance for washing and/or drying clothes comprising an outer cabinet (2) provided with an opening (8) for loading and unloading clothes and a door (9) for closing the opening (8), a drum (14) rotatably supported inside said cabinet (2), an opening (19) provided in said drum (14) in correspondence to the opening (8) of the cabinet (2), and a flexible member (18) connecting the opening (8) of the cabinet (2) to the opening (19) of the drum (14) with watertightness, **characterised in that** the flexible member (18) comprises a polymeric material and at least one fragrance compound mixed in at least a portion of said polymeric material.

2. Household appliance according to claim 2, **characterised in that** said at least one fragrance compound is either natural or synthetic.

3. Household appliance according to claim 3, **characterised in that** said polymeric material has a hardness of about Shore A 25-55.

4. Method for manufacturing a flexible member adapted to connect with watertightness an opening for loading and unloading clothes in a cabinet of a washer and/or dryer household appliance to an the opening of a drum rotatably supported inside said cabinet, **characterised in that** it comprises:
providing a polymeric material,
moulding said polymeric material,
adding at least one fragrance compound into said polymeric material during said moulding phase;
curing said material with said at least one fragrance therein.

5. Household appliance for washing and/or drying clothes comprising an outer cabinet (2) provided with an opening (8) for loading and unloading clothes and a door (9) for closing the opening (8), a drum (14) rotatably supported inside said cabinet (2), an opening (19) provided in said drum (14) in correspondence to the opening (8) of the cabinet (2), and a flexible member (18) connecting the opening (8) of the cabinet (2) to the opening (19) of the drum (14) with watertightness, **characterised in that** said flexible member (18) is adapted to removably house at least one tablet including at least one fragrance compound.

6. Household appliance according to claim 5, **characterised in that** said flexible member (18) comprises at least one pocket for removaby housing said tablet.

7. Household appliance according to claim 5 o 6, **characterised in that** said tablet comprises a polymeric material and at least one fragrance compound mixed in at least a portion of said polymeric material.
